# EUROPÄISCHE PATENTSCHRIFT

(11) **EP 0 976 743 B1**
(45) Veröffentlichungstag und Bekanntmachung des Hinweises auf die Patenterteilung: **17.10.2001**
(21) Anmeldenummer: 99114393.4
(22) Anmeldetag: 22.07.1999
(51) Int. Cl.: C07D 323/06

(54) **Abtrennung von Trioxan aus gasförmigen Gemischen mit Formaldehyd**
Separation of trioxanne from gas mixtures with formaldehyde
Séparation de trioxanne à partir de mélanges gazeux avec du formaldéhyde

(30) Priorität: 25.07.1998 DE 19833620
(43) Veröffentlichungstag der Anmeldung: 02.02.2000
(73) Patentinhaber: Ticona GmbH, 65451 Kelsterbach (DE)
(72) Erfinder: Kniep, Hagen, Dr., 65931 Frankfurt (DE); Meister, Christine, 65843 Sulzbach (DE); Schweers, Elke, Dr., 69812 Bad Soden (DE); Nicolaou, Ioannis, Dr., 65779 Kelkheim (DE); Scheid, Dirk, 65529 Waldems (DE)

(56) Entgegenhaltungen:
- DE-A- 3 508 668
- GB-A- 1 245 990

## Beschreibung

Die Erfindung betrifft ein Verfahren zur Trennung eines gasförmigen Gemisches enthaltend Formaldehyd und Trioxan.

Zur Herstellung von technischen Kunststoffen wie Polyacetalen bzw. Polyoxymethylen wird hochreines Trioxan benötigt. Die Qualität des Kunststoffes, speziell der Polymerisationsgrad, wird neben den Polymerisationsbedingungen vorrangig durch die Reinheit des Trioxan bestimmt.

Es sind verschiedene Verfahren zur Herstellung von Trioxan bekannt: z. B. homogene oder heterogene saure Katalyse wäßriger Formaldehydlösungen - aus der AT 252913 - oder heterogene Gasphasen - Katalyse an Heteropolysäuren - aus der EP 0606056. Unabhängig vom Herstellverfahren fällt Trioxan nicht als reiner Stoff, sondern im Gemisch mit Formaldehyd und geringen Mengen anderer Komponenten an (z. B. Methanol, Wasser, Methylformiat, Methylal, Ameisensäure, Dioxolan, Tetroxan). Zur Verwendung des Trioxans in der Polymerisation wird dieses insbesondere vom Formaldehyd abgetrennt und sollte nur geringe Mengen an Nebenkomponenten enthalten.

Es ist eine Vielzahl von Literatur bekannt, um wäßrige Gemische aus Formaldehyd und Trioxan zu trennen. Die Trennung gasförmiger Gemische bestehend aus o. g. Komponenten wurde nur in wenigen Stellen beschrieben.

Die Trennung aus wäßriger Lösung erfolgte bisher insbesondere über die Rektifikation (AT 252913 (D1)). Bei der Rektifikation wird nicht umgesetzter Formaldehyd häufig wieder in den Reaktor zurückgeführt und dort weiter zu Trioxan umgesetzt, wodurch der Umsatz der Reaktion bezogen auf den Formaldehyd erhöht wird. Ein Grenzwert für die Rektifikation ergibt sich durch ein bei 92 °C und 1 bar siedendes Azeotrop mit Wasser, welches in unterschiedlichen Mengen im Ausgangsgemisch vorhanden sein kann.

Zur Vermeidung von Feststoffbildung durch Polymerisation von reinem Formaldehyd bzw. der Bildung von para-Formaldehyd müssen alle Apparateteile, die mit reinem Formaldehyd in Berührung kommen, entweder auf Temperaturen > 100 °C (bei 1 bar Formaldehyd-Partialdruck) aufgeheizt oder mit einer Flüssigkeit benetzt werden. Dies betrifft bei der Rektifikation insbesondere den Kopfkondensator und stellt einen großen Nachteil gegenüber Trennungen dar, die nur in der Flüssigphase ablaufen.

Ein alternatives Verfahren zur Trennung von Formaldehyd und Trioxan aus wäßrigen Lösungen besteht in der Extraktion des Trioxans mit organischen Lösungsmitteln, in denen Trioxan eine höhere physikalische Löslichkeit besitzt als der Formaldehyd. Diese Verfahren wurden ausschließlich zur Abtrennung des Trioxans aus der wäßrigen Phase eingesetzt. Als organische Extraktionsmittel wurden beispielsweise gesättigte aliphatische oder aromatische Kohlenwasserstoffe oder Halogenkohlenwasserstoffe (EP 0583907 (D2)) verwendet, die wenig oder nicht mit Wasser mischbar sind. Ein Nachteil der Extraktion ist, daß teilweise auch große Teile des Trioxans in der wäßrigen Phase verbleiben. Große Mengen müssen daher im Kreis geführt werden oder gehen beim Aufarbeitungsverfahren verloren.

Für die Trennung von gasförmigen Gemischen von Formaldehyd und Trioxan wurde mehrfach die selektive Absorption einer Spezies eingesetzt. Dabei wurde entweder der Formaldehyd i. a. chemisorbiert und das Trioxan in der Gasphase belassen (GB 12459903 (D3)) oder es erfolgte umgekehrt eine selektive Physisorption des Trioxans (EP 0680959 (D4)). Da keine flüssige Phase gefunden wurde, in der entweder nur der Formaldehyd oder nur das Trioxan löslich ist, werden auch Anteile der jeweils anderen Spezies gebunden. Dadurch können mit diesem Verfahren nicht die für die Polymerisation nötigen Reinheiten erzielt werden. Weiterhin entstehen große Verluste am Wertstoff Trioxan.

Als weitere Möglichkeit der selektiven Trennung aus einer wäßrigen Phase wurde die Kristallisation des Trioxans beschrieben. Dieses Verfahren wurde zur Abtrennung des Trioxans aus wäßrigen Formaldehyd - Trioxan - Gemischen eingesetzt (DE 3508668 (D5)). Wobei aber die Trioxankonzentration in dem wäßrigen Gemisch größer als 50 Gew.-% sein mußte.

Vor diesem Stand der Technik lag der Erfindung die Aufgabe zugrunde, ein Verfahren bereitzustellen, das eine Abtrennung von Trioxan aus einem gasförmigen Gemisch vorzugsweise mit Formaldehyd ermöglicht, wobei die nachteilige irreversible Bildung von festem para-Formaldehyd vermieden werden sollte.

Die bezüglich der Trioxan - Formaldehyd - Trennung unspezifischen Gas-Flüssigkeits-Gleichgewichte bei der Absorption sollten aufgrund der zu erwartenden geringen Ausbeuten nicht zur Trennung genutzt werden.

Diese Aufgabe wird erfindungsgemäß dadurch gelöst, daß man aus dem Gemisch den Formaldehyd und das Trioxan mindestens teilweise in einer alkoholhaltigen Flüssigkeit löst und das Trioxan aus der somit erhaltenen Lösung kristallisiert und abtrennt.

Gegenstand der Erfindung ist daher ein Verfahren zur Trennung eines gasförmigen Gemisches enthaltend Formaldehyd und Trioxan, dadurch gekennzeichnet, daß man aus dem Gemisch den Formaldehyd und das Trioxan mindestens teilweise in einer alkoholhaltigen Flüssigkeit löst und das Trioxan aus der somit erhaltenen Lösung kristallisiert und abtrennt.

Bevorzugte Ausführungsformen sind in den Unteransprüchen offenbart.

Das gasförmige Gemisch kann zu mindestens 50 Gew.-% aus Formaldehyd (FA) und Trioxan (TOX) bestehen und bei Konzentrationen nahe 100 Gew.-% (FA+TOX) ggf. nur geringe Mengen an Nebenkomponenten enthalten. Es wird in geeigneter Weise mit einer alkoholhaltigen Flüssigkeit, vorzugsweise einem Alkohol, der ein-oder mehrwertig sein kann, behandelt, d.h. ein Anteil an Trioxan und Formaldehyd von mindestens 50 % wird bei Temperaturen im Bereich von -20 bis 100 °C, vorzugsweise im Bereich von 20 bis 80 °C, gelöst, d.h. sorbiert. Besonders bevorzugt wird mindestens 80% und ganz besonders bevorzugt nahezu der gesamte Anteil, d.h. mehr als 95 % gelöst. Als Nebenkomponenten im gasförmigen Gemisch können beispielsweise Wasser, Methanol, Methylformiat, Tetroxan, Dioxolan und Spuren von Ameisensäure auftreten. Durch das Lösen des Gases in flüssigem Alkohol wird die Bildung von festem para-Formaldehyd wirkungsvoll unterdrückt. Die Absorptionskapazität für Formaldehyd ist aufgrund einer bekannten Chemisorption (Bildung eines Hemiformal) nahezu unbegrenzt. Die Abtrennung des TOX, welches physisorbiert wird, kann im erfindungsgemäßen Verfahren anschließend durch eine Kristallisation bei Temperaturen im Bereich von -20 bis 63 °C, vorzugsweise im Temperaturbereich von -10 bis 55 °C erfolgen.

Das erfindungsgemäße Verfahren löst im Gegensatz zu D5 die Aufgabe der Trennung des gasförmig und wasserarm vorliegenden Gemisches enthaltend Formaldehyd und Trioxan, wie es nach der Trimerisierung von wasserarmem Formaldehyd entsteht. Es bildet damit einen bedeutenden Schritt in einem Prozeß zur Herstellung von Trioxan aus Methanol, bestehend aus den Schritten nicht oxidative Dehydrierung (DE 3920811), Formaldehydtrimerisierung (EP 0606056, EP 0691388) und Trioxanabtrennung (hier). Da der Vorteil des neuen Prozesses insgesamt die wasserarme Herstellung von Trioxan ist, stellt D5 hier keine brauchbare Lösung dar.

Im Unterschied zu D3 und D4 können beim erfindungsgemäßen Verfahren sowohl Formaldehyd als auch Trioxan vollständig absorbiert werden, wobei vorzugsweise ein einwertiger Alkohol, z. B. Cyclohexanol. Methanol, Propanol, Butanol verwendet wird. Es ist aber auch der Einsatz anderer. auch mehrwertiger Alkohole (z. B. Glyzerol, di-Ethylen-Glykol, tri-Ethylen-Glykol, tri-Ethanolamin, Butantriol, Pentantriol) möglich.

Das erfindungsgemäße Verfahren zeichnet sich dabei gegenüber den bisher eingesetzten Verfahren durch folgende Vorteile aus:
- keine Feststoffbildung durch para-Formaldehyd aufgrund der Chemisorption des Formaldehyd/Trioxan - Gemisches in flüssiger Phase,
- hohe Absorptions-Kapazität aufgrund des als Absorptionsmittel eingesetzten Alkohols sowohl für Formaldehyd als auch für Trioxan,
- günstige Verhältnisse von im Kreis geführten Trioxanmengenströmen zum ausgeschleusten Mengenstrom,
- hohe Reinheiten und Ausbeuten an Trioxan durch Kristallisation erzielbar.
- günstiges Verhältnis von eingesetzter Energie zur gewonnen Trioxanmenge.

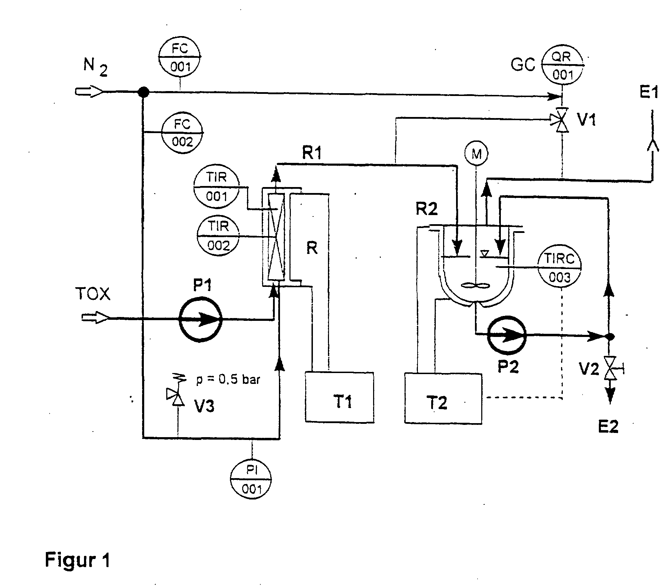

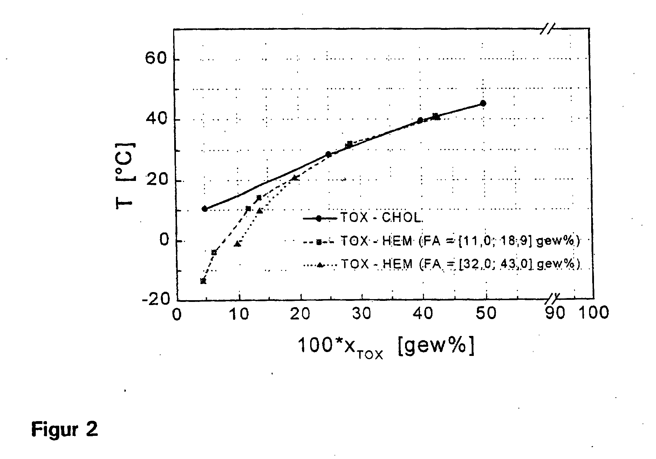

Das erfindungsgemäße Verfahren sei nachfolgend anhand einiger experimenteller Untersuchungen und der Figuren 1 und 2 illustriert. Eine Beschränkung in irgendeiner Weise ist dadurch nicht beabsichtigt.

Es zeigt:
- Fig. 1:: ein Verfahrensfließbild zur Versuchsanlage für die durchgeführten Experimente;
- Fig. 2:: Erstarrungskurven für Trioxan in Cyclohexanol bzw. Cyclohexyl-Hemiformal mit unterschiedlichen Formaldehydgehalten.

Zur Untersuchung der Absorption des gasförmigen Gemisches aus Formaldehyd und Trioxan wurden Versuche mit der in Fig. 1 skizzierten Apparatur durchgeführt. Über eine Pumpe P1 wird flüssiges Trioxan (TOX) mit einem Trägergasstrom aus Stickstoff vermischt und in einen Spaltreaktor R gefördert. In diesem wird das Trioxan verdampft und in der Gasphase heterogen katalysiert an Heteropolysäuren (VO(HPO)₄) zu Formaldehyd gespalten. Der dabei erreichte Umsatz kann über die Temperatur in weiten Grenzen eingestellt werden, womit auch das Verhältnis von Trioxan und Formaldehyd am Reaktorausgang eingestellt werden kann. Das derart erzeugte gasförmige Gemisch wird über beheizte Leitungen R1 in einen Rührkessel R2 geleitet, welcher mit 0,5 kg Cyclohexanol gefüllt ist. Der Kessel wird mittels Begasungsrührer mit einer Drehzahl von 2000 min⁻¹ gerührt und über einen Doppelmantel bei unterschiedlichen Temperaturen thermostatisiert. Der Flüssigkeitsinhalt wird ferner über eine Umpumpeinrichtung P2 geleitet, um aus diesem Kreislauf Proben zur Analyse der Flüssigphase entnehmen zu können. Die Zusammensetzung der Gasphase erfolgte on-line mittels eines Gaschromatographen GC.

Die Parameter und Ergebnisse der zur Absorption durchgeführten Versuche sind in nachstehender Tabelle 1 zusammengefaßt. Dabei wurden unterschiedliche Zusammensetzungen des aufgegebenen Gasstromes als auch unterschiedliche Temperaturen der Flüssigphase. d. h. des vorgelegten Alkohols, untersucht. Die Ergebnisse zeigen, daß ein großer Teil (80 bis 95 %) des eingesetzten Massenstromes an Formaldehyd als auch an Trioxan absorbiert wird.

**Tab. 1**

| Ergebnisse der Versuche zur Absorption des gasförmigen Gemisches aus Formaldehyd (FA) und Trioxan (TOX). | | | | | | | |
|---|---|---|---|---|---|---|---|
| Nr. | T [°C] | Konzentr. FA ein [Gew.-%] | Konzentr. TOX ein [Gew.-%] | Konz. N₂ [Gew.-%] | Verhältnis FA : TOX | Absorption FA [%] | Absorption TOX [%] |
| 1A | 30 | 31,2 | 51,5 | 17,3 | 0,61 | 97,3 | 95,7 |
| 2A | 50 | 64,2 | 13,8 | 22,0 | 4,65 | 96,5 | 65,1 |
| 3A | 80 | 30,6 | 49,8 | 19.6 | 0,62 | 80,5 | 90,1 |

Aus Tab. 1 ist ersichtlich, daß sich bei den Versuchen für steigende Temperaturen T der Flüssigphase die absorbierte Gasmenge verringert. Der im Gleichgewicht sorbierte Anteil an zugeführtem Formaldehyd und Trioxan ist um so höher, je tiefer die Temperatur ist und je höher die Konzentration dieser Komponenten ist.

Im erfindungsgemäßen Verfahren kann die Absorption kontinuierlich oder diskontinuierlich, bevorzugt jedoch kontinuierlich durchgeführt werden. Die zu sorbierenden Gase können im Gleich- oder Gegenstrom mit dem Absorbens geführt werden. Zur apparativen Umsetzung dieses Teilschrittes des erfindungsgemäßen Verfahrens können Rührkessel, Kolonnen mit verschiedenen Einbauten (z. B. Packungen, Füllkörper, Böden) oder Blasensäulen verwendet werden.

Die Bildung trioxanreicher Feststoffe infolge der Kristallisation kann im gleichen Apparat erfolgen wie die Absorption, bevorzugt sind jedoch diese beiden Schritte getrennt durchzuführen. Der Druck ist für die Feststoffbildung unkritisch. Die Temperatur der Flüssigphase ist möglichst gering zu wählen, um die Absorption zu begünstigen (vgl. Tab. 2), bei der apparativen Trennung von Absorption und Kristallisation jedoch so hoch, daß für eine gegebene Trioxankonzentration noch kein Trioxan auskristallisiert. Der Zusammenhang zwischen Temperatur und der Trioxan-Konzentration, bei der das Trioxan gerade zu erstarren beginnt, wird durch die Erstarrungskurve oder Liquiduslinie angegeben.

In weiteren durchgeführten Versuchen wurde das Ausfallen von Trioxan als Feststoff aus Lösungen aus Cyclohexanol bzw. Cyclohexyl-Hemiformal (gebildet durch Chemisorption von Formaldehyd in Cyclohexanol) und Trioxan untersucht. Es zeigte sich, daß der Verlauf der Liquiduslinie im System Trioxan - Alkohol/Formaldehyd bzw. Trioxan - Hemiformal auch durch die Konzentration des Formaldehyds bestimmt wird. Als Ergebnis dieser Versuche sind drei gemessene Liquiduskurven jeweils für unterschiedliche Formaldehyd-Konzentrationen in Fig. 2 gezeigt.

Fig. 2 verdeutlicht, daß für steigende Formaldehyd-Konzentrationen im Alkohol bzw im Hemiformal die Liquiduslinie zu geringeren Temperaturen verschoben wird.

Beispielhaft wurden zur Kristallisation des Trioxans aus einer Cyclohexyl-Hemiformallösung weitere Versuche durchgeführt. Dazu wurde das entsprechende Stoffgemisch in eine thermostatisierte Röhre, wie sie standardmäßig zur Untersuchung der Kristallisation verwendet wird, gefüllt. Mittels eines programmierbaren Thermostaten wurde die Manteltemperatur in unterschiedlichen Gradienten verändert.

Dabei wurde so vorgegangen, daß ein Gemisch aus Trioxan und Cyclohexanol / Cyclohexyl-Hemiformal als Ausgangslösung mit einem linearen Temperaturgradienten abgekühlt wurde Nach Erreichen einer Endtemperatur wurde die restliche Mutterlauge abgelassen. Dann wurde das Kristallisationsrohr langsam aufgeheizt und bei unterschiedlichen Temperaturen abgeschmolzenes Schwitzöl, das bei Erwärmung aus dem Kristallisat abschmilzt, abgelassen. Zum Ende des Versuches wurden alle restlichen Kristalle abgeschmolzen. Diese Restschmelze wird im folgenden als Endfraktion bezeichnet.

Nachfolgend sind die Ergebnisse von beispielhaft durchgeführten Versuche in kurzer Form erläutert.

### •VERSUCH 1 K

Im ersten Versuch wurden 210,43 g einer Ausgangslösung (24,8 Gew.-% Trioxan, 26,4 Gew.-% Formaldehyd, 47,7 Gew.-% Cyclohexyl-Hemiformal, Rest Methanol, Wasser) mit einem linearen Temperaturgradienten von -0,1 K/min von 30 °C auf -5 °C abgekühlt. Die Temperatur von -5 °C wurde für einen längeren Zeitraum gehalten und schließlich wurde die Mutterlauge abgelassen.

Das im Gefäß verbliebene Gemisch wurde dann bei konstanter Aufheizgeschwindigkeit von 0,11 K/min auf 65 °C aufgeheizt. Zu diskreten Zwischenzeiten wurde die dabei abgeschwitzte Flüssigkeit in mehreren Zwischenfraktionen abgezogen. Bei 65 °C war das Restgemisch vollständig aufgeschmolzen und wurde als Endfraktion abgelassen.

Der Trioxangehalt in der Endfraktion betrug 93,8 Gew.-%. Es wurden 51,4 % der eingesetzten Trioxanmenge in der Endfraktion wiedergefunden. Der Formaldehydgehalt in der Endfraktion betrug 2,3 Gew.-%.

### •VERSUCH 2K:

In einem zweiten Versuch wurden 179,3 g einer Ausgangslösung (24,8 Gew.-% Trioxan, 26,4 Gew.-% Formaldehyd, 47.7 Gew.-% Cyclohexyl-Hemiformal, Rest Methanol, Wasser) mit einer Abkühlgeschwindigkeit von -0,05 K/min von 40 °C auf -10 °C abgekühlt. Die Temperatur von -10 °C wurde für einen längeren Zeitraum gehalten und schließlich wurde die Mutterlauge abgelassen.

Das im Gefäß verbliebene Gemisch wurde dann mit einer Aufheizgeschwindigkeit von 0,16 K/min auf 65 °C aufgeheizt. Bei verschiedenen Temperaturen wurden zu bestimmten Zeiten die dabei abgeschwitzten Flüssigkeitsvolumina in Zwischenfraktionen abgezogen. Bei 65 °C war das Restgemisch vollständig aufgeschmolzen und wurde als Endfraktion abgelassen.

Der Trioxangehalt in der Endfraktion betrug 52,5 Gew.-%. Es wurden 83,2 % der eingesetzten Trioxanmenge in der Endfraktion wiedergefunden. Der Formaldehydgehalt in der Endfraktion betrug 17,0 Gew.-%.

### •VERSUCH 3K:

In einem dritten Versuch wurden 144,7 g einer Ausgangslösung (26,2 Gew.-% Trioxan, 26,3 Gew.-% Formaldehyd, 47,2 Gew.-% Cyclohexyl-Hemiformal, Rest Methanol, Wasser) mit einer Abkühlgeschwindigkeit von 0,1 K/min von 25 °C auf -5 °C abgekühlt. Die Temperatur von -5 °C wurde für einen längeren Zeitraum gehalten und schließlich wurde die Mutterlauge abgelassen.

Das im Gefäß verbliebene Gemisch wurde dann mit einer Aufheizgeschwindigkeit von 0,6 K/min auf 65 °C aufgeheizt. Zu diskreten Zwischenzeiten wurde die dabei abgeschwitzte Flüssigkeit in mehreren Zwischenfraktionen abgezogen. Bei 65 °C war das Restgemisch vollständig aufgeschmolzen und wurde als Endfraktion abgelassen.

Der Trioxangehalt in der Endfraktion betrug 97,7 Gew.-%. Es wurden 25,6 % der eingesetzten Trioxanmenge in der Endfraktion wiedergefunden. Der Formaldehydgehalt in der Endfraktion betrug 0,8 Gew.-%.

### •VERSUCH 4K (gerührt):

In einem vierten Versuch wurden 50,0 g einer Ausgangslösung (35,2 Gew.-% Trioxan, 16,0 Gew.-% Formaldehyd. 48,1 Gew.-% Cyclohexyl-Hemiformal, Rest Methanol, Wasser) in einem Doppelmantelgefäß mit Magnetrührer gerührt. Über den Doppelmantel wurde die Ausgangslösung mit 0,2 K/min von 35 °C auf 10 °C abgekühlt. Das Trioxan kristallisierte dabei als nadelförmiges Kristallisat aus. Aus der Suspension wurden sehr kleine Probemengen mikroskopisch untersucht und der Durchmesser der Kristallnadeln zu ca. 100 µm bestimmt. Die Länge war unregelmäßig.

Die gesamte Suspension wurde schließlich bei der Temperatur von 10 °C aus dem Rührgefäß abgelassen und über eine Fritte, an deren Filtratseite ein Vakuum von 200 mbar angelegt wurde, filtriert:

Das Filtrat (Mutterlauge) und der Filterkuchen (Kristallfraktion) wurden anschließend analysiert. Der Trioxangehait in der Kristallfraktion betrug 76,8 Gew.-%. Der Formaldehydgehalt in der Kristallfraktion betrug 5,8 Gew.-%. In der Mutterlauge wurden 19,1 % des eingesetzten Trioxans wiedergefunden. Damit verblieben 80,9 % des eingesetzten Trioxans in der Kristallfraktion.

### •VERSUCH 5K (gerührt):

In einem fünften Versuch wurden 1000,0 g einer Ausgangslösung (35,7 Gew.-% Trioxan, 16,8 Gew.-% Formaldehyd, 46,8 Gew.-% Cyclohexyl-Hemiformal. Rest Methanol, Wasser) in einem Doppelmantelgefäß mit Magnetrührer bei 200 min⁻¹ gerührt. Über den Doppelmantel wurde die Ausgangslösung mit 0,2 K/min von 33 °C auf 5 °C abgekühlt. Das Trioxan kristallisierte dabei als nadelförmiges Kristallisat aus. Aus der Suspension wurden sehr kleine Probemengen mikroskopisch untersucht und der Durchmesser der Kristallnadeln zu ca. 100 µm bestimmt. Die Länge war unregelmäßig.

Die gesamte Suspension wurde schließlich bei der Temperatur von 5 °C aus dem Rührgefäß abgelassen und in einer Siebbecherzentrifuge filtriert.

Das Filtrat (Mutterlauge) und der Filterkuchen (Kristallfraktion) wurden anschließend analysiert. Der Trioxangehalt in der Kristallfraktion betrug 85,9 Gew.-%. Der Formaldehydgehalt in der Kristallfraktion betrug 3,5 Gew.-%. In der Mutterlauge betrug die Konzentration des Trioxans 9.2 Gew.-%.

Im Sinne einer kontinuierlichen Prozeßführung bietet sich vorzugsweise eine Modifikation der in den Versuchen 4 und 5 beschriebenen Durchführung an. Als Rührapparat ist hierfür beispielsweise ein Kratzkühler geeignet, der auf den Wänden angewachsene Kristalle ständig abkratzt und so den Wärmeübergang zur Wand begünstigt. Der Kratzkühler kann vorteilhaft mit einem adiabat betriebenen Rührkessel kombiniert werden, in dem die Kristalle aus der im Kratzkühler unterkühlten Flüssigkeit aufwachsen können.

In die Kombination aus Kratzkühler und adiabatischem Rührkessel wird kontinuierlich flüssige auf Bedingungen oberhalb der Liquiduslinie eingestellte Ausgangslösung eingespeist und am Ausgang der Apparatur kontinuierlich eine Suspension mit Trioxankristallen entzogen.

Aus der abgezogenen Suspension wird mittels Fest-Flüssig-Trennverfahren der Feststoff (Trioxankristalle) abgetrennt. Als Fest-Flüssig-Trennverfahren können beispielsweise Filternutschen, Bandfilter, Zentrifugen aber auch beliebige andere Verfahren eingesetzt werden.

### Bezugszeichenliste zu Figur 1:

- P1: Pumpe zur Förderung von TOX und N₂
- P2: Umwälzpumpe
- R: Spaltreaktor
- R1: beheizte Leitung
- R2: Rührkessel
- T1, T2: Thermostat
- V1, V2, V3: Ventil/Absperrhahn
- E1: Abzug
- E2: Probenahme

## Patentansprüche

1. Verfahren zur Trennung eines gasförmigen Gemisches enthaltend Formaldehyd und Trioxan, **dadurch gekennzeichnet, daß** man aus dem Gemisch den Formaldehyd und das Trioxan mindestens teilweise in einer alkoholhaltigen Flüssigkeit löst und das Trioxan aus der somit erhaltenen Lösung kristallisiert und abtrennt.

2. Verfahren nach Anspruch 1, **dadurch gekennzeichnet, daß** der Anteil an Formaldehyd und Trioxan im Gemisch mindestens 50 Gew.-% beträgt und daß man den Anteil zu mindestens 50 % in der alkoholhaltigen Flüssigkeit löst.

3. Verfahren nach Anspruch 1, **dadurch gekennzeichnet, daß** das Lösen von Formaldehyd und Trioxan in einer alkoholhaltigen Flüssigkeit bei Temperaturen im Bereich von -20 bis +100 °C erfolgt.

4. Verfahren nach Anspruch 1, **dadurch gekennzeichnet, daß** die alkoholhaltige Flüssigkeit als Lösungsmittel mindestens einen ein- oder mehrwertigen Alkohol enthält.

5. Verfahren nach Anspruch 1, **dadurch gekennzeichnet, daß** die Kristallisation des Trioxans bei Temperaturen im Bereich von -25 °C bis 63 °C erfolgt.

6. Verfahren nach Anspruch 1 oder 5. **dadurch gekennzeichnet, daß** die Kristallisation des Trioxans durch Abkühlung bei stehender Lösung erfolgt.

7. Verfahren nach Anspruch 1 oder 5. **dadurch gekennzeichnet, daß** das Trioxankristallisat an gekühlten Wänden gebildet wird, während die Lösung umgepumpt wird.

8. Verfahren nach Anspruch 1 oder 5, **dadurch gekennzeichnet, daß** das Trioxankristallisat unter Rühren der Lösung in Form einer Suspension erzeugt wird.

9. Verfahren nach Anspruch 8. **dadurch gekennzeichnet**. daß das Trioxankristallisat mittels einer Sedimentation im Erdschwerefeld oder im Fliehkraftfeld abgetrennt wird.

10. Verfahren nach Anspruch 8. **dadurch gekennzeichnet, daß** das 5 Trioxankristallisat mitteis einer Filtration abgetrennt wird.

## Claims

1. A method for separating a gaseous mixture comprising formaldehyde and trioxane, wherein at least some of the formaldehyde and the trioxane are dissolved from the mixture in a alcohol-containing liquid and the trioxane is crystallized from the solution thus obtained and is separated.

2. The method as claimed in claim 1, wherein the proportion of formaldehyde and trioxane in the mixture is at least 50 wt% and wherein at least 50% of said proportion is dissolved in the alcohol-containing liquid.

3. The method as claimed in claim 1, wherein the dissolution of formaldehyde and trioxane in an alcohol-containing liquid takes place at temperatures in the range of from -20 to +100°C.

4. The method as claimed in claim 1, wherein the alcohol-containing liquid contains as a solvent at least one mono- or polyhydric alcohol.

5. The method as claimed in claim 1, wherein the crystallization of the trioxane takes place at temperatures in the range from -25° C to 63 °C.

6. The method as claimed in claim 1 or 5, wherein the crystallization of the trioxane takes place by cooling while the solution is at rest.

7. The method as claimed in claim 1 or 5, wherein the trioxane crystallizate is formed at cold walls while the solution is recycled.

8. The method as claimed in claim 1 or 5, wherein the trioxane crystallizate is generated in the form of a suspension while the solution is stirred.

9. The method as claimed in claim 8, wherein the trioxane crystallizate is separated off by means of sedimentation in the gravity field of the Earth or in a centrifugal force field.

10. The method as claimed in claim 8, wherein the trioxane crystallizate is separated off by means of a filtration.

## Revendications

1. Procédé de séparation d'un mélange gazeux contenant du formaldéhyde et du trioxanne, **caractérisé en ce que** l'on dissout hors du mélange le formaldéhyde et le trioxanne au moins en partie dans un liquide contenant de l'alcool et que l'on cristallise et sépare le trioxanne de la solution ainsi obtenue.

2. Procédé selon la revendication 1, **caractérisé en ce que** la proportion de formaldéhyde et de trioxanne dans le mélange est d'au moins 50% en poids et que l'on dissout la proportion à raison d'au moins 50% dans le liquide contenant de l'alcool.

3. Procédé selon la revendication 1, **caractérisé en ce que** la dissolution du formaldéhyde et du trioxanne se fait dans un liquide contenant de l'alcool à des températures dans le domaine allant de -20 à +100°C.

4. Procédé selon la revendication 1, **caractérisé en ce que** le liquide contenant de l'alcool contient, en tant que solvant, au moins un alcool mono- ou polyhydrique.

5. Procédé selon la revendication 1, **caractérisé en ce que** la cristallisation du trioxanne se fait à des températures dans le domaine allant de -25°C à 63°C.

6. Procédé selon la revendication 1 ou 5, **caractérisé en ce que** la cristallisation du trioxanne se fait par refroidissement avec la solution au repos.

7. Procédé selon la revendication 1 ou 5, **caractérisé en ce que** le produit de cristallisation de trioxanne se forme sur des parois refroidies, pendant le pompage de la solution.

8. Procédé selon la revendication 1 ou 5, **caractérisé en ce que** le produit de cristallisation de trioxanne est produit par agitation de la solution sous la forme d'une suspension.

9. Procédé selon la revendication 8, **caractérisé en ce que** le produit de cristallisation de trioxanne est séparé dans le champ de pesanteur ou dans le champ de forces centrifuges à l'aide d'une sédimentation.

10. Procédé selon la revendication 8, **caractérisé en ce que** le produit de cristallisation de trioxanne est séparé à l'aide d'une filtration.
